(19) Europäisches Patentamt / European Patent Office / Office européen des brevets

(11) **EP 4 389 914 A1**

(12) **EUROPEAN PATENT APPLICATION**

(43) Date of publication:
**26.06.2024 Bulletin 2024/26**

(21) Application number: **22216339.6**

(22) Date of filing: **23.12.2022**

(51) International Patent Classification (IPC):
*C12Q 1/6818* (2018.01)    *C12Q 1/6804* (2018.01)

(52) Cooperative Patent Classification (CPC):
(C-Sets available)
**C12Q 1/6818; C12Q 1/6804**     (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **CSEM Centre Suisse d'Electronique et de**
**Microtechnique SA - Recherche et Développement**
**2002 Neuchâtel (CH)**

(72) Inventor: **PORCHETTA, Alessandro**
**00169 Rome (IT)**

(74) Representative: **Bovard SA Neuchâtel**
**Rue des Beaux-Arts 8**
**2000 Neuchâtel (CH)**

Remarks:
The complete document including Reference Table(s) and the Sequence Listing(s) can be downloaded from the EPO website

(54) **CRISPR-CAS BASED METHOD FOR THE DETECTION OF NON-NUCLEIC ACID TARGETS**

(57) The present invention relates to a target detection system, and a method adapted from the trans-cleavage activity of a CRISPR-Cas system for detecting a target molecule in a sample. The present invention is furthermore related to a kit comprising said detection system, the use of said detection system as a diagnostic agent, and a diagnostic device.

**FIG.6**

EP 4 389 914 A1

(52) Cooperative Patent Classification (CPC): (Cont.)

C-Sets
**C12Q 1/6804, C12Q 2521/301, C12Q 2525/161,**
**C12Q 2525/301, C12Q 2537/101,**
**C12Q 2537/1373, C12Q 2537/157,**
**C12Q 2537/162, C12Q 2563/131,**
**C12Q 2565/1015;**
**C12Q 1/6818, C12Q 2521/301, C12Q 2525/161,**
**C12Q 2525/301, C12Q 2537/101,**
**C12Q 2537/1373, C12Q 2537/157,**
**C12Q 2537/162, C12Q 2563/131,**
**C12Q 2565/1015**

**Description**

**TECHNICAL FIELD**

The present invention relates to a target detection system, and a method adapted from the trans-cleavage activity of a CRISPR-Cas system for detecting a target in a sample. The present invention is furthermore related to a kit comprising said detection system, the use of said detection system as a diagnostic agent, and a diagnostic device.

**BACKGROUND OF THE INVENTION**

Technologies based on clustered regularly interspaced short palindromic repeats (CRISPR) and associated proteins (Cas) have revolutionized genome editing. Specifically, Cas enzymes are endonucleases that are able to cleave the phosphodiester bond within a polynucleotide chain upon specific target DNA binding to the complementary crRNA. This activity is known as cis-cleavage activity. Of note, a protospacer adjacent motif (PAM) is a short DNA sequence, usually 2-6 base pairs in length, that follows the DNA region targeted for cleavage by the CRISPR system. The PAM is generally required for a Cas nuclease to cut and is generally found 3-4 nucleotides downstream from the cut site.

Some Cas enzymes such as Cas12, Cas13 and Cas14 also show an additional collateral a-specific nuclease activity known as trans-cleavage activity that is activated upon target DNA/RNA binding. This property has been widely exploited to develop biosensing platforms. More recently, potent analytical chemistry tools for ultrasensitive DNA and RNA detection have been elaborated, enabling impressive advances in nucleic acid diagnostics thanks to collateral indiscriminate cleavage activity of specific Cas enzymes, the trans-cleavage activity. However, to date only few examples of CRISPR-Cas-based sensing methods have been reported for the detection of non-nucleic acid targets by using the CRISPR-Cas systems.

Recently, a new peptide display platform has been developed adapted from the genome editing technology CRISPR that can be used as a tool to identify antibodies in patient blood samples (Barber K. W. at al., Molecular Cell. 2021, 81(17), 3650-8). The platform has been employed to detect antibodies binding to proteins derived from pathogens by creating a DNA-templated self-assembling peptide microarray. This platform has been adapted for sensing purposed of antibodies in serum sample. However, this platform does not provide any quantitative information and can be used only for screening purposes. In addition, the detection system employs peptide libraries fused to catalytically inactive Cas9 and so does not exploit the trans-cleavage nuclease activity to generate catalytic output.

Proximity-driven strategies of CRISPR-Cas systems for detection of diverse molecular analytes, including antibodies and small molecules have been described in PCT/US2020/030032. The sensing platform takes advantage of collateral ssDNase or ssRNase activity of Cas enzymes activated by the simultaneous co-localization of two antigen-labelled DNA/RNA strands. Specifically, the first ssDNA and the second ssDNA sequences comprise complementary sticky ends and form a double-stranded protospacer adjacent motif (PAM) sequence when the first and second synthetic nucleotide sequences are brought into proximity by binding of the first and second antigens to a target molecule. However, the method described required multiple steps with many different reagents such as a ligase, and a polymerase.

Therefore, there is a need for a detection system for detecting with high specificity a target in a sample by a simple method adapted from the trans-cleavage activity of a CRISPR-Cas system that would be very effective using only few reagents.

**SUMMARY OF THE INVENTION**

The present invention provides a detection system for detecting the presence of a target molecule (13) in a sample comprising

   a) a first oligonucleotide comprising a first hairpin structure comprising

      i. a first 5' single stranded overhang region (2) comprising at least 5 nucleotides;

      ii. a first double stranded stem region (1) comprising at least 16 base pairs, and 0 mismatch or at least 1 mismatch, and wherein said first double stranded stem region (1) comprises a target nucleotides sequence complementary to a guide sequence of a crRNA (11) of a CRISPR-Cas system; and

      iii. a first single stranded loop region (3) comprising from 1 to 4 nucleotides complementary to a protospacer-adjacent motif (PAM) capable of being recognized by the CRISPR-Cas system;

   b) a second oligonucleotide, comprising

i. a second 5' single stranded region (4) comprising the PAM capable of being recognized by the CRISPR-Cas system;

ii. a second single stranded region (6) complementary to the target nucleotides sequence of the first double stranded stem region (1); and

iii. a second single stranded region (7) linked to a terminal recognition element (5) capable of being recognized by the target molecule (13);

and

c) a third oligonucleotide, comprising

i. a third 5' single stranded region (9) linked to a terminal recognition element (5) capable of being recognized by the target molecule (13), wherein said third 5' single stranded region (9) is partially complementary to the second single-stranded region (7); and

ii. a third single stranded region (8) complementary to the first 5' single-stranded overhang region (2).

The invention further relates to the use of a detection system according to the present invention as a diagnostic agent.

The invention also provides a kit comprising

a) a detection system or a diagnostic device according to the invention; and
b) instructions for use to detect the presence of a target molecule (13) in a sample.

The invention also relates to a method for detecting a target molecule (13) in a sample, the method comprising:

a) providing a first oligonucleotide comprising a first hairpin structure comprising

i. a first 5' single stranded overhang region (2) comprising at least 5 nucleotides;

ii. a first double stranded stem region (1) comprising at least 16 base pairs, and 0 mismatch or at least 1 mismatch, and wherein said first double stranded stem region (1) comprises a target nucleotides sequence complementary to a guide sequence of a crRNA (11) of a CRISPR-Cas system; and

iii. a first single stranded loop region (3) comprising from 1 to 4 nucleotides complementary to a protospacer-adjacent motif (PAM) capable of being recognized by the CRISPR-Cas system;

b) providing a second oligonucleotide, comprising

i. a second 5' single stranded region (4) comprising the PAM capable of being recognized by the CRISPR-Cas system;

ii. a second single stranded region (6) complementary to the target nucleotides sequence of the first double stranded stem region (1); and

iii. a second single stranded region (7) linked to a terminal recognition element (5) capable of being recognized by the target molecule (13);

c) providing a third oligonucleotide, comprising

i. a third 5' single stranded region (9) linked to a terminal recognition element (5) capable of being recognized by the target molecule (13), wherein said third 5' single stranded region (9) is partially complementary to the second single-stranded region (7); and

ii. a third single stranded region (8) complementary to the first 5' single-stranded overhang region (2);

d) providing a sample comprising the target molecule (13);

e) incubating said sample comprising the target molecule (13) with the first oligonucleotide, the second oligonucleotide, and the third oligonucleotide;

thereby, in the presence of the target molecule (13) that binds to the terminal recognition element (5) of the second single-stranded region (7) and third 5' single stranded region (9), allowing said second oligonucleotide and said third oligonucleotide to bind to the first oligonucleotide, by triggering its reconfiguration and to form a complex;

f) incubating the reaction from step e) with

    i. a CRISPR-Cas system comprising a Cas enzyme (10), and the crRNA (11) comprising a guide sequence complementary to the first double stranded stem region (1); and

    ii. a DNA reporter construct (12);

thereby, allowing the guide sequence of the crRNA (11) to bind to the complex of step e), thereby allowing the Cas enzyme (10) to cleave the reporter construct (12); and

g) determining the presence of the target molecule (13) in the sample by measuring signal gain values.

**BRIEF DESCRIPTION OF THE FIGURES**

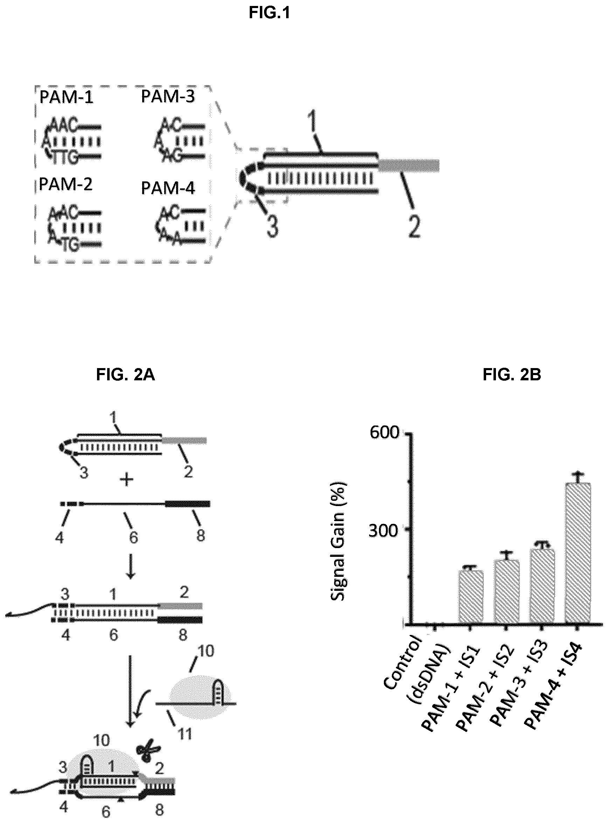

Figure 1 illustrates first oligonucleotides (PAM-1, PAM-2, PAM-3, and PAM-4) comprising a first 5' single stranded overhang region (2), a first double stranded stem region (1) and a first single stranded loop region (3) comprising 1 nucleotide (PAM-1), 2 nucleotides (PAM-2), 3 nucleotides (PAM-3), and 4 nucleotides (PAM-4), complementary to a protospacer-adjacent motif (PAM).

Figure 2: (A) illustrates a mechanism to activate (switch ON) Cas12-based trans-cleavage activity using an invading DNA strand (IS, 4+6+8) and a strand displacement-like reaction. The reconfiguration of the DNA hairpin translator results in PAM complementation (3 binds to 4). This results in a consequent recognition of the crRNA/Cas12 complex (10+11) and activation of Cas12 trans-cleavage activity; (B) represents Fluorescence signal gain values (%) obtained in the presence of saturating concentrations (100 nM) of invading DNA strands (IS1, IS2, IS3, IS4) complementary respectively to specific 1st oligonucleotides (PAM-1, PAM-2, PAM-3, and PAM-4). "Control dsDNA" refers to a standard double stranded DNA containing the same PAM motif already accessible to Cas12 binding.

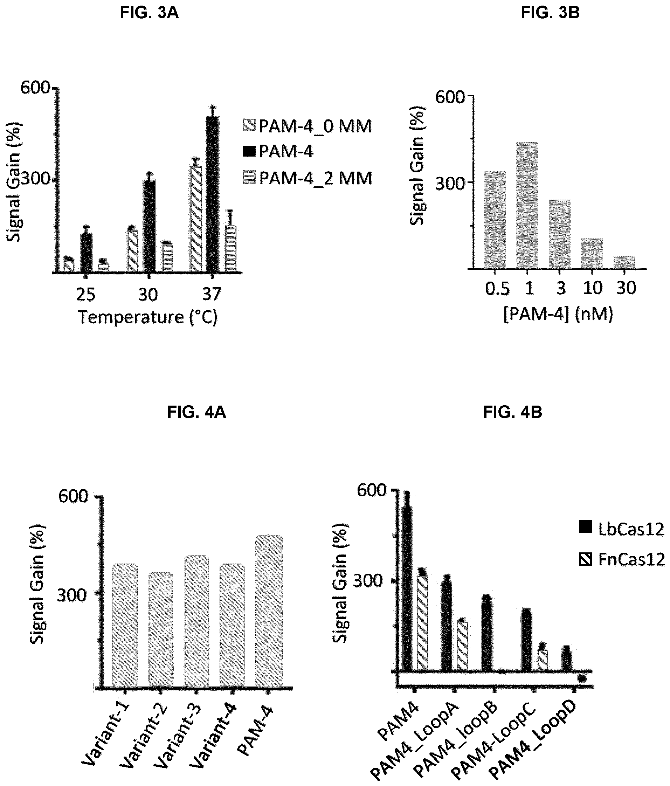

Figure 3: (A) shows the effect of temperature over Cas12 trans cleavage activity induced by reconfiguration of a set of different first oligonucleotides having different stem stability (0 mismatch, PAM-4_0MM; 1 mismatch, PAM-4; 2 mismatch, PAM-4_2MM) in the presence of invading strand (IS-4 at 100nM); (B) Fluorescence signal gain obtained in the presence of different concentrations of the 1st oligonucleotide (PAM-4) in solution by adding the complementary IS-4 (100 nM, composed by region number 4+6+8).

Figure 4: (A) Fluorescence signal gain obtained using different 1st oligonucleotides variant -1, -2, -3, -4 and PAM-4 having different sequences in the stem region (B) Fluorescence signal gain obtained using different 1st oligonucleotides with different bases in the loop (region "3") but the same stem region, in the presence of saturating concentrations of the corresponding complementary invading DNA strand.

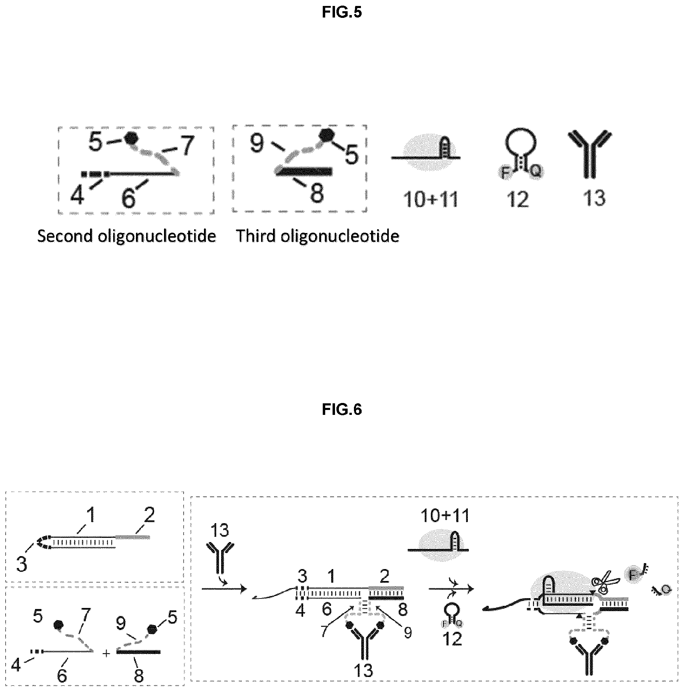

Figure 5: illustrates some components of the detection system used in the method of the invention.

Figure 6: is a schematic description of the Cas-based sensing platform for antibody detection.

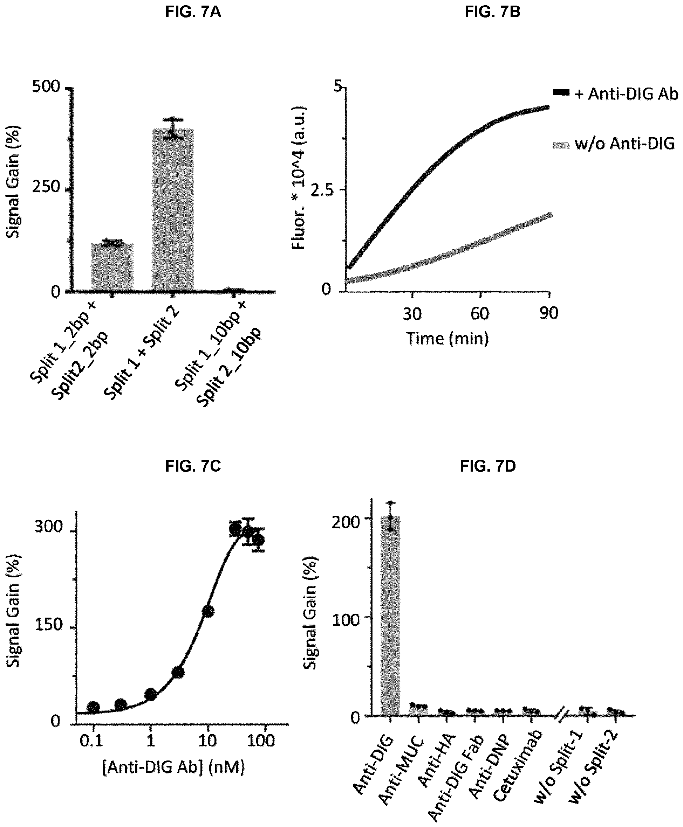

Figure 7: (A) Fluorescence signal gain obtained using 2nd (split 1) and 3rd oligonucleotides (split -2) having different complementary region (2bp, 6bp and 10bp); (B) Kinetic profiles obtained by adding 100 nM of Anti-DIG Ab in a solution containing all the sensing elements compared to the kinetic profile of the reaction mixture in the absence of Ab; (C) Fluorescence signal gain obtained when detecting the presence of the Anti-DIG Ab at different concentration in the sample (0.1, 1, 10 and 100nM); (D) Fluorescence signal gain obtained with the recognition element (DIG) showing the specificity of the method performed on samples with different IgG Ab. A control has been performed in the presence of either the 2nd oligonucleotide (Split 1) or the 3rd oligonucleotide (Split 2).

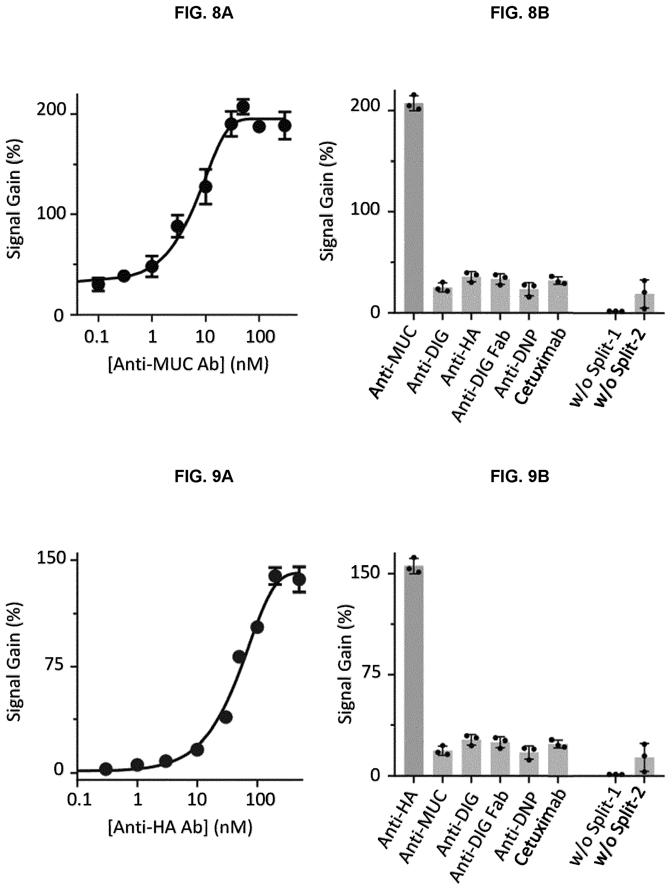

Figure 8: (A) Fluorescence signal gain obtained when detecting the presence of the Anti-MUC Ab at different concentration in the sample (0.1, 1, 10 and 100nM); (B) Fluorescence signal gain obtained with the recognition element (MUC) showing the specificity of the method performed on samples with different IgG Ab. A control has been performed in the presence of either the 2nd oligonucleotide (Split 1) or the 3rd oligonucleotide (Split 2).

Figure 9: (A) Fluorescence signal gain obtained when detecting the presence of the Anti-HA Ab at different concentration in the sample (0.1, 1, 10 and 100nM); (B) Fluorescence signal gain obtained with the recognition element (HA) showing the specificity of the method performed on samples with different IgG Ab. A control has been performed in the presence of either the 2nd oligonucleotide (Split 1) or the 3rd oligonucleotide (Split 2).

## DETAILED DESCRIPTION OF THE INVENTION

The present invention relates to a detection system for detecting the presence of a target molecule (13) in a sample comprising

   a) a first oligonucleotide comprising a first hairpin structure comprising

      i. a first 5' single stranded overhang region (2) comprising at least 5 nucleotides;

      ii. a first double stranded stem region (1) comprising at least 16 base pairs, and 0 mismatch or at least 1 mismatch, and wherein said first double stranded stem region (1) comprises a target nucleotides sequence complementary to a guide sequence of a crRNA (11) of a CRISPR-Cas system; and

      iii. a first single stranded loop region (3) comprising from 1 to 4 nucleotides complementary to a protospacer-adjacent motif (PAM) capable of being recognized by the CRISPR-Cas system;

   b) a second oligonucleotide, comprising

      i. a second 5' single stranded region (4) comprising the PAM capable of being recognized by the CRISPR-Cas system;

      ii. a second single stranded region (6) complementary to the target nucleotides sequence of the first double stranded stem region (1); and

      iii. a second single stranded region (7) linked to a terminal recognition element (5) capable of being recognized by the target molecule (13);

   and

   c) a third oligonucleotide, comprising

      i. a third 5' single stranded region (9) linked to a terminal recognition element (5) capable of being recognized by the target molecule (13), wherein said third 5' single stranded region (9) is partially complementary to the second single-stranded region (7); and

      ii. a third single stranded region (8) complementary to the first 5' single-stranded overhang region (2).

The following definitions are supplied to facilitate the understanding of the present invention.

As used herein, the term "comprise" is generally used in the sense of include, that is to say permitting the presence of one or more features or components.

As used herein, the singular form "a", "an" and "the" include plural references unless the context clearly dictates otherwise.

As used herein, an "oligonucleotide" refers to a sequence of DNA residues that form a molecule. They can be found as single stranded DNA (ssDNA) molecule, or as double stranded DNA (dsDNA) molecule. ssDNA and dsDNA can be linked together to form complex structures. Oligonucleotides can be linear, but also exist as stem-loop structure.

As used herein, a "stem-loop" refers to intramolecular base pairing that can occur in single-stranded DNA when two regions of the same strand, usually complementary in nucleotide sequence when read in opposite directions, form a double helix that ends in an unpaired loop. The structure is also known as a "hairpin" or "hairpin loop". Some oligonucleotides adopt a stem-loop structure depending on the stability of the resulting DNA helix and DNA loop region. The stability of the helix is determined by its length, the number of mismatches it contains and the base composition of the paired region.

As used herein, a "mismatch" (MM) is a DNA defect occurring when two non-complementary bases are aligned in a base-pair of a double stranded DNA.

As used herein, a "crRNA" comprises a guide sequence complementary to a region in a target DNA sequence, such as the first double stranded stem region (1) in the context of the present invention; and comprises a sequence recognized by a Cas enzyme of a CRISPR-Cas system.

As used herein, a "guide sequence of a crRNA" is a sequence complementary to a region in a target DNA sequence, such as the first double stranded stem region (1) in the context of the present invention.

As used herein, a "protospacer-adjacent motif" (PAM) is a short DNA sequence in a target DNA sequence recognized by a Cas enzyme of a CRISPR-Cas system. This motif is required for a Cas nuclease to cut the target DNA sequence.

As used herein, a "reporter DNA construct" refers to a polynucleotide-based molecule that can be cleaved by an activated CRISPR-associated (Cas) enzyme with a trans-cleavage activity to produce a detectable signal or a detectable molecule.

As used herein, a "Cas enzyme" is a nuclease directed to a specific target DNA sequence by a guide sequence of a crRNA, where the Cas enzyme makes a double-strand break in the target DNA sequence. In particular, the Cas enzyme has a protospacer-adjacent motif (PAM) dependent nuclease activity.

As used herein, a "recognition element" is a molecule for which a target molecule has high affinity. Depending on the target molecule, the recognition element is for example an antigen, i.e. a small molecule, a peptide, a protein such as an antibody, and/or a DNA/RNA aptamer.

As used herein, a target molecule is a molecule that may be in a sample and that specifically binds through non-covalent bonds to a recognition element. The target molecule will be detected in the sample using the detection system. The target molecule is a non-nucleic acid molecule. It may be a peptide, a protein having multimeric domains such as an antibody, a receptor, or an enzyme. For example, it is possible to detect DNA repair enzymes whose catalytic activity can provide a means to control the folding stability of the first oligonucleotide containing the bases complementary to a protospacer-adjacent motif (PAM).

As used herein, the term "linked" indicates a direct or an indirect link between molecules where directly linked molecules comprise covalent bonds between two molecules.

In the present invention, the first oligonucleotide comprises a first 5' single stranded overhang region (2) comprising at least 5 nucleotides. Preferably, the first 5' single stranded overhang region (2) comprises 5 to 30, 5 to 20, or 5 to 10 nucleotides, and more preferably, 10 nucleotides.

The first oligonucleotide further comprises a first double stranded stem region (1) comprising at least 16 base pairs, wherein said region comprises a target nucleotides sequence complementary to a guide sequence of a crRNA (11) of a CRISPR-Cas system. Preferably, the first double stranded stem region (1) comprises 16 to 24, or 16 to 20 base pairs, and more preferably, 20 base pairs.

The first double stranded stem region (1) may comprise 0 mismatch or at least 1 mismatch. Preferably, the first double stranded stem region (1) comprises 0, 1, 2, 3 or 4 mismatches, and more preferably, 1 mismatch. In the example part, it has been demonstrated that the detection of the signal values is improved when using a first double stranded stem region (1) comprising one mismatch (Figure 3A).

The first oligonucleotide further comprises a first single stranded loop region (3) comprising from 1 to 4 nucleotides complementary to a protospacer-adjacent motif (PAM) capable of being recognized by the CRISPR-Cas system. Preferably, the first single stranded loop region (3) comprises 4 nucleotides complementary to a protospacer-adjacent motif (PAM) capable of being recognized by the CRISPR-Cas system. In the example part, it has been demonstrated that the detection of the signal values is improved when using a first single stranded loop region (3) comprising 4 nucleotides complementary to a protospacer-adjacent motif (PAM) capable of being recognized by the CRISPR-Cas system (Figure 1 and 2B).

Preferably, the sequence of the first single stranded loop region (3) is selected from the group comprising 5'-CAAA-3'; 5'-CAAG-3'; 5'-CAGA-3'; and/or 5'-CGAA-3'. More preferably, the sequence of the first single stranded loop region (3) is 5'-CAAA-3'. In the example part, it has been demonstrated that signal values are detectable when using a first single stranded loop region (3) comprising 5'-CAAA-3'; 5'-CAAG-3'; 5'-CAGA-3'; and/or 5'-CGAA-3'. In addition, the detection of the signal is improved when using a first single stranded loop region (3) comprising 5'-CAAA-3' (Figure 4B).

According to the present invention, the sequence of the first oligonucleotide may be selected from the group comprising SEQ ID NO: 1, 2, 3, 4, 5, 6, 7, 8, 9, and/or 10. Preferably, the sequence of the first oligonucleotide is SEQ ID NO: 4.

The second oligonucleotide comprises a second 5' single stranded region (4) comprising the PAM capable of being recognized by the CRISPR-Cas system. The PAM capable of being recognized by the CRISPR-Cas system may be selected from 5'-T-3'; 5'-TT-3'; 5'-TTT-3'; 5'-TTTG-3'; 5'-CTTG-3'; 5'-GTCT-3'; 5'-GCTT-3'; 5'-GCCC-3'; and/or 5'-GTTT-3'. Preferably, the PAM is 5'-TTTG-3'.

The second oligonucleotide further comprises a second single stranded region (6) complementary to the first double stranded stem region (1); and a second single stranded region (7) linked to a terminal recognition element (5) capable of being recognized by the target molecule (13).

The second single region (7) may be directly linked to the terminal recognition element (5), with covalent bonds between the second single region (7) and the terminal recognition element (5). Alternatively, the second single region (7) may be indirectly linked to the terminal recognition element (5) through an additional strand (ssDNA, a locked nucleic acid, or a RNA molecule) complementary to the second single region (7) and wherein said additional strand is linked to the terminal recognition element (5).

According to the present invention, the sequence of the second oligonucleotide may be selected from the group comprising SEQ ID NO; 15 and/or 17. Preferably the sequence of the second oligonucleotide is SEQ ID NO: 17.

In the present invention, the third oligonucleotide comprises a third 5' single stranded region (9) linked to a terminal recognition element (5) capable of being recognized by a target molecule (13).

The third 5' single stranded region (9) may be directly linked to the terminal recognition element (5), with covalent bonds between the third 5' single stranded region (9) and the terminal recognition element (5). Alternatively, the third 5' single stranded region (9) may be indirectly linked to the terminal recognition element (5) through an additional strand (ssDNA, a locked nucleic acid, or a RNA molecule) complementary to the third 5' single stranded region (9) and wherein said additional strand is linked to the terminal recognition element (5).

The third 5' single stranded region (9) is partially complementary to the second single-stranded region (7). At least 2 nucleotides are complementary in the nucleotide sequence when read in opposite directions between both regions and form a double stranded DNA. Preferably, the third 5' single stranded region (9) comprises 2, 3, 4, 5, 6, 7, 8 and 9 nucleotides complementary to the second single-stranded region (7). More preferably, the third 5' single stranded region (9) comprises 6 nucleotides complementary to the second single-stranded region (7).

In the example part, it has been demonstrated that signal gain values are detectable when using a complementary region from 2 nucleotides or 6 nucleotides. The detection of signal gain values has been improved when using a complementary region of 6 nucleotides (Figure 7A)

According to the present invention, the sequence of the third oligonucleotide may be selected from the group comprising SEQ ID NO: 16, and /or 18. Preferably, the sequence of the third oligonucleotide is SEQ ID NO: 18.

In the present invention, the second and third oligonucleotides may be linked to one or more recognition elements (5) independently selected from the group comprising a small molecule, a peptide and/or a protein such as an antibody. In particular, the terminal recognition element (5) of the second and third oligonucleotides may be identical or different.

In the present invention, the detection system may further comprise

d) a CRISPR-Cas system comprising a Cas enzyme (10), and the crRNA (11) comprising a guide sequence complementary to the first double stranded stem region (1); and/or

e) a DNA reporter construct (12).

A CRISPR-Cas system comprises a Cas enzyme and a crRNA, whereby the Cas enzyme may bind to the crRNA. The CRISPR-Cas system may bind to a region of a target DNA sequence that is complementary to the guide sequence of the crRNA.

In the present invention, the CRISPR-Cas system is adapted to bind to the first oligonucleotide comprising a first double stranded stem region (1) upon reconfiguration of the first oligonucleotide.

The Cas enzymes useful for the systems and methods of the present invention possess trans-cleavage activity. In the present invention, the Cas enzyme (10) is activated following binding to the first oligonucleotide upon reconfiguration of the first oligonucleotide. The Cas enzyme (10) remains active and continues to non-specifically cleave non-target oligonucleotides, such as DNA reporter constructs (12).

In the present invention, it is necessary that CRISPR-Cas systems exhibit a non-specific collateral cleavage activity of single-stranded nucleic acids such as DNA reporter constructs together with a specific DNA target sequence recognition referred as trans-cleavage activity.

In the present invention, any Cas enzyme having a trans-cleavage activity and a non-specific collateral cleavage activity of single-stranded nucleic acids may be used.

For example, the Cas enzyme (10) may be selected from the group comprising Cas 12, Cas $\phi$ and/or Cas 14. Preferably, the Cas enzyme (10) is Cas12a (Cpf1) such as Lba Cas 12a or FnCas12a.

In the present invention, it is necessary that the first double stranded stem region (1) be complementary to a guide sequence of the crRNA (11), for being recognized by the Cas enzyme (10) of a CRISPR Cas system.

The guide sequence of the crRNA (11) comprises a region of at least 16 to 24, or 16 to 20 ribonucleotides complementary to the first double stranded stem region (1). More preferably, the guide sequence of the crRNA (11) comprises 20 ribonucleotides complementary to the first double stranded stem region (1).

Preferably, the crRNA (11) is SEQ ID NO: 21.

By designing DNA reporter constructs (12), the trans-cleavage activity of the Cas enzyme (10) can be monitored with fluorescence-based and lateral flow-based assays or any other reporter systems indicative of the presence or absence of a target molecule in the sample. In the present invention, the DNA reporter construct (12) may be selected from the group comprising a fluorophore quencher stem-loop oligonucleotide, a methylene blue stem-loop oligonucleotide, and/or a ferrocene stem-loop oligonucleotide immobilized on an electrode.

Preferably, the DNA reporter construct (12) is a fluorophore quencher stem-loop oligonucleotide wherein a first end is linked to a fluorophore; and a second end is linked to a quencher of the fluorophore. The fluorophore quencher stem-loop oligonucleotide is configured to be cleaved by the Cas enzyme (10), such that the signal can be produced following binding of the Cas enzyme (10) to the first oligonucleotide upon reconfiguration of the first oligonucleotide.

The detection systems of the invention may be used to detect target molecules in a sample such as molecules that specifically bind through non-covalent bonds to a recognition element (5). Target molecules may be a peptide, a protein having multimeric domains such as but not limited to an antibody, a receptor, or an enzyme, such as a DNA repair enzyme whose catalytic activity can provide a means to control the folding stability of the first oligonucleotide containing the bases complementary to a protospacer-adjacent motif (PAM).

Thus, the present invention also relates to an antibody detection system comprising

a) a first oligonucleotide comprising a first hairpin structure comprising

i. a first 5' single stranded overhang region (2) comprising at least 5 nucleotides;

ii. a first double stranded stem region (1) comprising at least 16 base pairs, and 0 mismatch or at least 1 mismatch, and wherein said first double stranded stem region (1) comprises a target nucleotides sequence complementary to a guide sequence of a crRNA (11) of a CRISPR-Cas system; and

iii. a first single stranded loop region (3) comprising from 1 to 4 nucleotides complementary to a protospacer-adjacent motif (PAM) capable of being recognized by the CRISPR-Cas system;

b) a second oligonucleotide, comprising

i. a second 5' single stranded region (4) comprising the PAM capable of being recognized by the CRISPR-Cas system;

ii. a second single stranded region (6) complementary to the target nucleotides sequence of the first double stranded stem region (1); and

iii. a second single stranded region (7) linked to a terminal recognition element (5) capable of being recognized by the antibody (13);

and

c) a third oligonucleotide, comprising

i. a third 5' single stranded region (9) linked to a terminal recognition element (5) capable of being recognized by the antibody (13), wherein said third 5' single stranded region (9) is partially complementary to the second single-stranded region (7); and

ii. a third single stranded region (8) complementary to the first 5' single-stranded overhang region (2).

The present invention also relates to a diagnostic device comprising at least one detection system according to the present invention, wherein the DNA reporter construct (12) is integrated on a screen-printed electrode.

The present invention also relates to the use of a detection system or a diagnostic device according to the present invention as a diagnostic agent.

The present invention also relates to a kit comprising

a) a detection system or a diagnostic device according to the present invention; and

b) instructions for use to detect the presence of a target molecule (13) in a sample.

The present invention also relates to a method for detecting a target molecule (13) in a sample, the method comprising:

a) providing a first oligonucleotide comprising a first hairpin structure comprising

i. a first 5' single stranded overhang region (2) comprising at least 5 nucleotides;

ii. a first double stranded stem region (1) comprising at least 16 base pairs, and 0 mismatch or at least 1 mismatch, and wherein said first double stranded stem region (1) comprises a target nucleotides sequence complementary to a guide sequence of a crRNA (11) of a CRISPR-Cas system; and

iii. a first single stranded loop region (3) comprising from 1 to 4 nucleotides complementary to a protospacer-adjacent motif (PAM) capable of being recognized by the CRISPR-Cas system;

b) providing a second oligonucleotide, comprising

i. a second 5' single stranded region (4) comprising the PAM capable of being recognized by the CRISPR-Cas system;

ii. a second single stranded region (6) complementary to the target nucleotides sequence of the first double stranded stem region (1); and

iii. a second single stranded region (7) linked to a terminal recognition element (5) capable of being recognized by the target molecule (13);

c) providing a third oligonucleotide, comprising

i. a third 5' single stranded region (9) linked to a terminal recognition element (5) capable of being recognized by the target molecule (13), wherein said third 5' single stranded region (9) is partially complementary to the second single-stranded region (7); and

ii. a third single stranded region (8) complementary to the first 5' single-stranded overhang region (2);

d) providing a sample comprising the target molecule (13);

e) incubating said sample comprising the target molecule (13) with the first oligonucleotide, the second oligonucleotide, and the third oligonucleotide;
thereby, in the presence of the target molecule (13) that binds to the terminal recognition element (5) of the second single-stranded region (7) and third 5' single stranded region (9), allowing said second oligonucleotide and said third oligonucleotide to bind to the first oligonucleotide, by triggering its reconfiguration and to form a complex;

f) incubating the reaction from step e) with

i. a CRISPR-Cas system comprising a Cas enzyme (10), and the crRNA (11) comprising a guide sequence complementary to the first double stranded stem region (1); and

ii. a DNA reporter construct (12);

thereby, allowing the guide sequence of the crRNA (11) to bind to the complex of step e), thereby allowing the Cas enzyme (10) to cleave the reporter construct (12); and

g) determining the presence of the target molecule (13) in the sample by measuring signal gain values.

As shown in the example part, the quantitative method is highly specific as different antibodies have been detected in samples depending on the specific recognition element (5) such as a small molecule or a peptide (Examples 5-7 and Figure 7D, 8B, 9B).

In one embodiment of the method of the invention, steps e) and f) are simultaneous.

In another embodiment of the method of the invention, step g) is performed using an optical, an electrochemical, a chemiluminescent and/or an electrochemiluminescent detection system.

In another embodiment of the method of the invention, the DNA reporter construct (12) is selected from the group comprising a fluorophore quencher stem-loop oligonucleotide, a methylene blue stem-loop oligonucleotide, and/or a ferrocene stem-loop oligonucleotide immobilized on an electrode.

In another embodiment of the method of the invention, step e) and/or f) are performed at a temperature of 15°C to 45°C, preferably, 37°C. As shown in the example part, signal gain values are detectable at 25°C, 30°C or 37°C when the sequence of the first oligonucleotide is SEQ ID NO: 4 (Example 2, Figure 3A).

EXAMPLES

Materials and methods

**Oligonucleotides:**

Oligonucleotides (HPLC purified) were purchased from Biosearch Technologies (Risskov, Denmark) and Metabion (Planegg, Germany). All oligonucleotides were dissolved in NE buffer (10 mM Tris-HCl / 50 mM NaCl/ 10 mM MgCl2, pH = 7.4) at a concentration of 100 μM and stored at -20°C. The concentration of the oligonucleotides was determined using Tecan Infinite M200pro (Männedorf, Switzerland) through NanoQuant Plate. Oligonucleotides were annealed before to use by thermocycler. Specifically, DNA oligonucleotides were incubated at 90°C for 2 min, RNA oligonucleotides at 65°C for 5 min, both slowly cooled to room temperature (1°C/min). The sequences of the oligonucleotides used are as follows.

First oligonucleotides

Table I

| Sequence name | Sequence |
|---|---|
| PAM-1 | **TCT TAC CCT ATC** *AGA TTT AGC CAT GTG TAG AC* CAA**A**TTG GT CTA CAC ATG <u>A</u>CT AAA TCT |
| PAM-2 | **TCT TAC CCT ATC** *AGA TTT AGC CAT GTG TAG AC* CA**AA**TG GT CTA CAC ATG <u>A</u>CT AAA TCT |
| PAM-3 | **TCT TAC CCT ATC** *AGA TTT AGC CAT GTG TAG AC* C <u>**AAA**</u> G GT CTA CAC ATG <u>A</u>CT AAA TCT |
| PAM-4 | **TCT TAC CCT ATC** *AGA TTT AGC CAT GTG TAG AC* <u>**CAAA**</u>GT CTA CAC ATG <u>A</u>CT AAA TCT |
| PAM-4_0 MM | **TCT TAC CCT ATC** *AGA TTT AGC CAT GTG TAG AC* <u>**CAAA**</u>GT CTA CAC ATG <u>G</u>CT AAA TCT |
| PAM_4_2MM | **TCT TAC CCT ATC** *AGA TTT AGC CAT GTG TAG AC* <u>**CAAA**</u>GT CTA CAC ATG <u>A</u>CT AAA T<u>G</u>T |

11

(continued)

| Sequence name | Sequence |
|---|---|
| PAM-4_loopA | **TCT TAC CCT ATC** *AGA TTT AGC CAT GTG TAG AC* **<u>CAAG</u>**GT CTA CAC ATG <u>A</u>CT AAA TCT |
| PAM-4_loopB | **TCT TAC CCT ATC** *AGA TTT AGC CAT GTG TAG AC* **<u>CAGA</u>**GT CTA CAC ATG <u>A</u>CT AAA TCT |
| PAM-4_loopC | **TCT TAC CCT ATC** *AGA TTT AGC CAT GTG TAG AC* **<u>CGAA</u>GT CTA CAC ATG <u>A</u>CT AAA TCT** |
| PAM-4_loopD | **TCT TAC CCT ATC** *AGA TTT AGC CAT GTG TAG AC* **<u>CGGG</u>**GT CTA CAC ATG <u>A</u>CT AAA TCT |
| Variant-1 | **TCT TAC CCT ATC** *TGTACTGCACTCGAGGCGTA***<u>CAAA</u>** TA CGC CTC GAG <u>A</u>GC AGT ACA |
| Variant-2 | **TCT TAC CCT ATC** *AGCACCCAGTCCGCCCTGAG***<u>CAAA</u>** CT CAG GGC GGA C<u>A</u>G GGT GCT |
| Variant-3 | **TCT TAC CCT ATC** *AGA TCT AGG GAT GTG TAG AC* **<u>CAAA</u>** GT CTA CAC ATC <u>A</u>CT AGA TCT |
| Variant-4 | **TCT TAC CCT ATC** *GGA TAT ACG CAT CTG TAG AC* **<u>CAAA</u>** GT TCA CAG ATG <u>A</u>GT ATA TCC |

In Table I, the first 5' single stranded overhang region (2) is represented by bold bases; one strand of the first double stranded stem region (1) of 20 nucleotides is represented by italic bases; the protospacer adjacent motif in the first single stranded loop region (3) is represented by bold and underlined bases; the complementary strand of the first double stranded stem region (1) is represented by normal bases with 20 nucleotides; An underlined normal base mismatch is introduced in the complementary strand of the first double stranded stem region (1).

Second oligonucleotides and third oligonucleotides

Table II

| | Sequence name | Sequence |
|---|---|---|
| Second oligonucleotide | Split 1_2bp | AGACTTTG *GT CTA CAC ATG GCT AAA TCT* **TT<u>C</u>A TTT TTT TTT TTT**-(Dig) |

(continued)

| | Sequence name | Sequence |
|---|---|---|
| Third oligonucleotide | Split 2_2bp | (Dig)-**TTT TTT TTT TTT <u>TGTT</u>**_GAT AGG GTA AGA_ |
| Second oligonucleotide | Split 1 | AGACTTTG _GT CTA CAC ATG GCT AAA TCT_ **TT<u>CAGACG</u> TTTTTTTTTTTT**-(Dig) |
| Third oligonucleotide | Split 2 | (Dig)-**TTTTTTTTTTTT<u>CGTCTG</u>TT** _GAT AGG GTA AGA_ |
| Second oligonucleotide | Split 1_10bp | AGACTTTG _GT CTA CAC ATG GCT AAA_ **TCT TT CAGACG ACAG TTTTTTTT**-(Dig) |
| Third oligonucleotide | Split 2_10bp | (Dig)-**TTTTTTTT <u>CTGT CGTCTG</u> TT** _GAT AGG GTA AGA_ |
| Second oligonucleotide | Split 1_HA | AGACTTTG _GT CTA CAC ATG GCT AAA_ _TCT_**T<u>CAGACG</u> TTTTTTTTTTTT**-(YPYDVPDYA) |
| Third oligonucleotide | Split 2_HA | (YPYDVPDYA)-**TTTTTTTTTTTT <u>CGTCTG</u> TT** _GAT AGG GTA AGA_ |
| Second oligonucleotide | Split 1_MUC | AGACTTTG _GT CTA CAC ATG GCT AAA TCT_ **TT<u>CAGACG</u> TTTTTTTTTTTT**- 3' – 3' - N-term APDTRPAPGSTAPPA - C-term – 5' |
| Third oligonucleotide | Split 2_MUC | 3' - N-term APDTRPAPGSTAPPA - C-term -5' **TTTTTTTTTTTT<u>CGTCTG</u>TT** _GAT AGG GTA AGA_ |

In Table II, with respect to the second oligonucleotides, the second single stranded region (6) (italic bases) is complementary to the first double stranded stem region (1) (italic bases) of the first oligonucleotide PAM-4; the second single-stranded region (7) (bold bases) comprises a 3' recognition element (5). The recognition element (5) is chemically labeled to the second oligonucleotide in 3'. It can be selected from a small molecule (e.g. DIG) or a peptide (e.g. MUC- and HA-associated recognition elements).

In Table II, with respect to the third oligonucleotides, the third single-stranded region (8) (italic bases) is complementary to the first 5' single stranded overhang region (2) (bold bases) of the first oligonucleotide PAM-4. The third 5' single stranded region (9) (bold bases) comprises a 5' recognition element (5). The recognition element (5) is chemically labeled to the third oligonucleotide in 5'. It can be selected from a small molecule (e.g. DIG) or a peptide (e.g. MUC- and HA-associated recognition elements).

Underlined bold bases are associated to complementary domains between the second and third oligonucleotide.

crRNA (11)

Table III

| Sequence name | Sequence |
|---|---|
| crRNA | 5'-UAA UUU CUACUAAGUGUAG AU<br><br>*G UC UAC ACA UGG CUA AAU CU* -3' |
| crRNA_Variant 1 | 5'UAAUUUCUACUAAGUGUAGAU *ACA UGA CGU GAG CUC CGC AU*-3' |
| crRNA-Variant 2 | 5'– UAAUUUCUACUAAGUGUAGAU *UCG UGG GUC AGG CGG GAC UC*-3' |
| crRNA-Variant 3 | 5'– UAAUUUCUACUAAGUGUAGAU *UCU AGA UCC CUA CAC AUC UG* -3' |
| crRNA-Variant 4 | 5'- UAAUUUCUACUAAGUGUAGAU *CCU AUA UGC GUA GAC AUC UG* |

In Table III, the portion of crRNA (11) complementary to the first double stranded stem region (1) of the first oligonucleotide is represented by italic bases.

**Materials:**

Sodium chloride (NaCl), magnesium chloride (MgCl2), tris(hydroxymethyl)aminomethane hydrochloride (Tris-HCl), 2-amino-2-(hydroxymethyl)-1,3-propanediol (Tris base), boric acid, ethylenediamine tetraacetic acid (EDTA), acrylamide/bis-acrylamide 30% solution, ammonium persulfate (APS), N,N,N',N'-tetramethyl ethylenediamine (TEMED), were purchased from Sigma-Aldrich, Italy and used without any further purifications. EnGen® Lba Cas12a from Lachnospiraceae bacterium ND2006 was purchased from New England Biolabs Inc. (Ipswich, UK). EnGen Lba Cas12a is a native protein expressed as a N-terminal 6-His-tagged fusion in E. coli. The enzyme has Simian virus 40 (SV40) T antigen nuclear localization sequences (NLSs) at both the N and C-termini of the protein. No mutations are present. FnCas12a from Francisella novocida bacterium was produced as reported in 1.3 Heterologous expression and purification of FnCas12a.

**Heterologous expression and purification of FnCas12a:**

The pDS015 plasmid, containing the Francisella novocida Cas12a gene was used for the heterologous expression of the protein in E. coli BL21(DE3) Rosetta™ 2 (Merck, Novagen), as fully described in the relative Bio-Protocol site (www.bio-protocol.org/e2842). Starting from 16 g of induced wet cells, we obtained ca. 10 mg of 95% purified protein, as confirmed by SDS-PAGE analysis. Aliquots of FnCas12a were finally stored at -20 °C.

**Fluorescence trans-cleavage assays:**

The experiments were performed in a 25 μL Tris buffer solution (10 nM Tris-HCl, 50 mM NaCl, 10 mM MgCl2, pH 7.4) containing the first oligonucleotide (1 nM), FRET-based reporter (500 nM), and either the invading strand (IS) in experiments of figures 2, 3 and 4, or the antigen-conjugated DNA strands (30 nM of each second and third oligonucleotide) and the specific target antibody in experiments of figures 6, 7, 8 and 9. After 15 min incubation at 37 ∘C, 2.5 ul of Cas12a/crRNA complex (200 nM), previously incubated for 30 min at 37∘C, were added to the sample and were immediately transferred to the PCR plate reader to record the kinetic fluorescence. The same experiments were carried out in the absence of the specific antibody or in the absence of invading strand in order to calculate signal gain. We performed all steady state fluorescence measurements using Agilent PCR. Kinetics experiments were performed by fixing excitation at $\lambda$exc = 488 nm and acquisition at $\lambda$em = 520 nm and setting the excitation and emission bandwidths to 5 nm in all the experiments. Signal gain fluorescence values were calculated according to the following equation:

$$\text{Signal Gain} = (\text{Fbound} - \text{Fbackground}) / \text{Fbackgroud} * 100$$

Where Fbound represents the fluorescence signal in the presence of the specific input (IS or target antibody) after 25 minutes, Fbackground is obtained from the same reaction mix in the absence of the specific input at the same time of analysis.

Example 1: Proposed mechanism to activate the Cas12-based trans-cleavage activity using an invading DNA strand (IS) and a strand displacement-like reaction.

Design of first oligonucleotides:

Figure 1 is a schematic representation of different first oligonucleotides PAM-1, PAM-2, PAM-3, PAM-4 comprising:

i. a first 5' single stranded overhang region (2) (Figure 1: region "2", toehold portion).

ii. a first double stranded stem region (1) (Figure 1: region "1)); and

iii. a first single stranded loop region (3) with different number of bases (Figure 1: region "3", loop from 1 to 4 nt); The bases in the loop are complementary to well-established PAM motifs, such as 5'-TTTG-3' that is complementary to PAM-4: 5'-CAAA-3'. The PAM motif must be complemented as a dsDNA through complementary base pairing with the loop of the first oligonucleotide to be recognized by the Cas12 enzyme.

The first oligonucleotides sequences are shown in Table I.

Design of invading strand (IS):

Figure 2A is a schematic representation of different invading strands (IS1, IS2, IS3, IS4) comprising:

i. a 5' single-stranded region (4) comprising the protospacer-adjacent motif PAM (Table IV: bold underlined bases) which is capable of being recognized by a CRISPR-Cas system; The PAM is complementary to the loop of the first single stranded loop region (3) of the first oligonucleotide;

ii. a single-stranded region (6) (Table IV: italic bases) complementary to the first double stranded stem region (1) of the first oligonucleotide; and

iii. a single-stranded region (8) (Table IV: bold bases) complementary to the first 5' single-stranded overhang region (2) of the first oligonucleotide.

The 4 extra bases at the 5' end of the IS are always present to facilitate the quantitative hairpin switch (see Table IV).

Table IV

| Sequence name | Sequence[a)] |
|---|---|
| IS-1 | CCAA<u>T</u>TTG *GT CTA CAC ATG GCT AAA TCT***GAT AGG GTA AGA** |
| IS-2 | ACCA<u>TT</u>TG *GT CTA CAC ATG GCT AAA TCT***GAT AGG GTA AGA** |
| IS-3 | GACC<u>TTT</u>G *GT CTA CAC ATG GCT AAA TCT* **GAT AGG GTA AGA** |

(continued)

| Sequence name | Sequence[a)] |
|---|---|
| IS-4 | AGAC**TTTG** *GT CTA CAC ATG GCT AAA TCT* **GAT AGG GTA AGA** |
| IS-4_LoopA | AGAC**CTTG** *GT CTA CAC ATG GCT AAA TCT* **GAT AGG GTA AGA** |
| IS-4_LoopB | AGAC**TCTG** *GT CTA CAC ATG GCT AAA TCT* **GAT AGG GTA AGA** |
| IS-4_LoopC | AGAC**TTCG** *GT CTA CAC ATG GCT AAA TCT* **GAT AGG GTA AGA** |
| IS-4_LoopD | AGAC**CCCG** *GT CTA CAC ATG GCT AAA TCT* **GAT AGG GTA AGA** |

As illustrated in figure 2A, the reconfiguration of the DNA hairpin first oligonucleotide results in PAM complementation by the binding of region (3) to region (4). This results in a consequent recognition of the crRNA/Cas12 complex (10+11) and activation of Cas12 trans-cleavage activity. When the crRNA/Cas12 complex (10+11) binds the complemented PAM and forms the R-loop structure, the nuclease activity is ON.

Fluorescence trans-cleavage assays have been performed according to the protocol described in materials and methods. Figure 2B represents the fluorescence signal gain obtained in the presence of saturating concentrations (100 nM) of invading DNA strands (IS1, IS2, IS3, IS4) complementary to the specific first oligonucleotides (PAM-1, PAM-2, PAM-3, PAM-4, respectively). The "Control dsDNA" refers to a standard double stranded DNA containing the same PAM motif already accessible to Cas12 binding. There is no hairpin, and in this case the system is already ON, so there is no signal change upon addition of complementary invading DNA strand (No signal gain for the control dsDNA on figure 2B).

The best signal gain % has been observed in presence of the first oligonucleotide PAM-4 and the invading strand IS4.

Example 2: Optimization of the sequence of the first oligonucleotide: stability of the first double stranded stem region (1)

Fluorescence trans-cleavage assays have been performed according to the protocol described in materials and methods.

Figure 3A shows the effect of temperature over Cas12 trans cleavage activity induced by reconfiguration of a set of different first oligonucleotides having different stem stability in the presence of invading strand (IS-4 at 100nM).

Tested first oligonucleotides have the same 4 bases sequestered in the loop but different stem stability. Specifically, different first nucleotides have been tested that present in the stem portion a different number of mismatches (0 mismatch, PAM-4_0MM; 1 mismatch, PAM-4; 2 mismatch, PAM-4_2MM) to evaluate the optimal signal gain as a function of hairpin stability (see Table I). In addition, different temperatures of incubation (25°C, 30°C and 37°C) of the reaction in presence of the CRISPR-Cas system have been tested.

Figure 3A shows that at all the tested temperatures, the first oligonucleotide PAM-4 having 1 mismatch in the first double stranded stem region (1) gives a better signal gain % compared to other tested first oligonucleotides having 0 or 2 mismatches (see table I, PAM-4_0MM, and PAM-4_2MM, respectively).

The first oligonucleotide PAM-4_0MM is too stable and partially switch in the presence of IS-4, whereas the first oligonucleotide PAM-4_2MM is unstable and is already partially switched in the absence of IS-4.

Figure 3B shows the fluorescence signal gain obtained in the presence of different concentrations of the first oligonucleotide PAM-4 in solution by adding the complementary IS-4 at 100 nM. Signal gain % are observed using PAM-4 concentrations at 0.5, 1, 3, 10 or 30 nM. Concentrations in the reaction of the first oligonucleotide between 0.5nM to 10nM give significant signal gain %. The best signal gain % is observed using the first oligonucleotide at a concentration of 1nM in the reaction.

Example 3: Optimization of the sequence of the first oligonucleotide: sequences of the stem region (1) and loop region (3).

Fluorescence trans-cleavage assays have been performed according to the protocol described in materials and methods.

Figure 4A shows the testing of the activation of Cas12 trans-cleavage activity upon reconfiguration induced by a complementary invading strand using different variants of the first oligonucleotide PAM-4.

Variants of PAM-4 (Variant -1, Variant -2, Variant -3, Variant -4; see Table I) have been tested with different sequences in the first double stranded stem region (1) (see Figure 1: "1" + its complementary portion) to demonstrate the generality of the approach of the present invention.

To activate the systems (switch ON), the corresponding invading strands IS (IS-1, IS-2, IS-3, IS-4) have been used at 100nM. Of note, for each variant, specific complementary crRNA (11) have been used (crRNA-Variant 1, crRNA-Variant 2, crRNA-Variant 3, crRNA-Variant 4, see Table III).

Figure 4A shows that all the different systems with the Variant-1, Variant-2, Variant-3, Variant-4 and PAM-4 can be switched ON in the presence of the invading strand. The signal gain % associated can be slightly different, in agreement with the sequence-dependence of the trans-cleavage activity of Cas12.

Figure 4B shows the fluorescence signal gain obtained using a set of first oligonucleotides derived from PAM-4 (PAM4-loopA, PAM-4-loopB, PAM-4-loopC, and PAM-4-loopD, see table I) with different bases in the first single stranded loop region (3) (region "3") but the same first double stranded stem region (1), in the presence of saturating concentrations of the corresponding complementary invading DNA strand.

The reactions have also been performed using two different Cas 12 enzymes: LbaCas12a or FnCas12a (see material and methods).

The best signal gain % has been observed in presence of LbaCas12a and the first oligonucleotide PAM4 having the loop motif 5'CAAA3', that is complementary to the PAM motif 5'TTTG3'.

Example 4: A Cas-12 based antibody detection system and method

Figure 5 is a schematic representation of different components of an antibody detection system according to the present invention as follows:

A second oligonucleotide comprising

    i. a second 5' single-stranded region (4) comprising the PAM capable of being recognized by the CRISPR-Cas system;

    ii. a second single stranded region (6) complementary to the target nucleotide sequence of the first double stranded stem region (1); and

    iii. a second single-stranded region (7) comprising a 3' recognition element (5) capable of being recognized by a target antibody (13). The 3' recognition element (5) can be for example a small molecule, a peptide, a protein, a synthetic recognition element, or an antibody. The region (7) is a DNA "linker" necessary to provide flexibility to the system. This domain can be partially complementary to the third 5' single stranded region (9) of the third oligonucleotide.

A third oligonucleotide comprising

    i. a third 5' single stranded region (9) comprising a 5' recognition element (5) capable of being recognized by the target antibody (13), wherein said third 5' single stranded region (9) is partially complementary to the second single-stranded region (7); The region (9) is a DNA "linker" necessary to provide flexibility to the system; and
    ii. a third single-stranded region (8) complementary to the first 5' single-stranded overhang region (2) of the first oligonucleotide. The region (8) is a DNA domain (about 10 nt) necessary to trigger the strand displacement reaction.

A first oligonucleotide has been represented on Figure 1 (Table I, Example I) comprising a first hairpin structure comprising

    i. a first 5' single stranded overhang region (2) comprising at least 5 nucleotides;

    ii. a first double stranded stem region (1) comprising at least 16 base pairs, and 0 mismatch or at least 1 mismatch, and wherein said first double stranded stem region (1) comprises a target nucleotides sequence complementary to a guide sequence of a crRNA (11) of a CRISPR-Cas system; and

iii. a first single stranded loop region (3) comprising from 1 to 4 nucleotides complementary to a protospacer-adjacent motif (PAM) capable of being recognized by the CRISPR-Cas system.

A Cas enzyme (10) having nuclease activity such as Cas 12.

A crRNA (11) that is complementary to the first double stranded stem region (1) of the first oligonucleotide. It is a mature CRISPR RNA incorporated into a Cas effector protein complex together with the Cas enzyme.

A DNA reporter construct (12) such as a FRET-based. In Figure 5, it is represented as a molecular beacon. Further DNA reporter constructs are also considered in the present invention, such as a short Fluorophore/Quencher modified DNA strand. Furthermore, in case of an electrochemical readout, the reporter will be tagged with an electrochemical label (e.g. methylen-blue, ferrocene) and anchored on an electrode surface.

Figure 6 is a schematic representation of the method of detection of a target molecule (13) that is an antibody using a detection system according to the present invention

The method comprises:

a) providing a first oligonucleotide comprising a first hairpin structure comprising

i. a first 5' single stranded overhang region (2) comprising at least 5 nucleotides;

ii. a first double stranded stem region (1) comprising at least 16 base pairs, and 0 mismatch or at least 1 mismatch, and wherein said first double stranded stem region (1) comprises a target nucleotides sequence complementary to a guide sequence of a crRNA (11) of a CRISPR-Cas system; and

iii. a first single stranded loop region (3) comprising from 1 to 4 nucleotides complementary to a protospacer-adjacent motif (PAM) capable of being recognized by the CRISPR-Cas system;

b) providing a second oligonucleotide, comprising

i. a second 5' single stranded region (4) comprising the PAM capable of being recognized by the CRISPR-Cas system;

ii. a second single stranded region (6) complementary to the target nucleotides sequence of the first double stranded stem region (1); and

iii. a second single stranded region (7) linked to a terminal recognition element (5) capable of being recognized by the target molecule (13);

c) providing a third oligonucleotide, comprising

i. a third 5' single stranded region (9) linked to a terminal recognition element (5) capable of being recognized by the target molecule (13), wherein said third 5' single stranded region (9) is partially complementary to the second single-stranded region (7); and

ii. a third single stranded region (8) complementary to the first 5' single-stranded overhang region (2);

d) providing a sample comprising the target molecule (13);

e) incubating said sample comprising the target molecule (13) with the first oligonucleotide, the second oligonucleotide, and the third oligonucleotide;
thereby, in the presence of the target molecule (13) that binds to the terminal recognition element (5) of the second single-stranded region (7) and third 5' single stranded region (9), allowing said second oligonucleotide and said third oligonucleotide to bind to the first oligonucleotide, by triggering its reconfiguration and to form a complex;

f) incubating the reaction from step e) with

i. a CRISPR-Cas system comprising a Cas enzyme (10), and the crRNA (11) comprising a guide sequence complementary to the first double stranded stem region (1); and

ii. a DNA reporter construct (12);

thereby, allowing the guide sequence of the crRNA (11) to bind to the complex of step e), thereby allowing the Cas enzyme (10) to cleave the reporter construct (12); and

g) determining the presence of the target molecule (13) in the sample by measuring signal gain values.

When the target molecule is an antibody, the Cas effector protein complex interrogates the complex of oligonucleotides formed between the first, second and third oligonucleotides in the presence of a target antibody. If the requirement for both sequence complementarity and a PAM site are satisfied, the Cas nuclease is activated, then the complex of oligonucleotides is cleaved in a site-specific way, and the reporter construct will be degraded by the Cas nuclease.

Example 5: Detection of the antibody Anti-DIG using a Cas-12 based antibody detection system

Fluorescence trans-cleavage assays have been performed according to the protocol described in materials and methods.

Figure 7A shows the effect of the complementary bases present in the second and third oligonucleotides (complementarity between region (7) and (9)).

Different second and third oligonucleotides sequences have been tested (Split 1_2bp + Split2_2bp; Split 1 + Split 2; and Split 1_10bp + Split 2_10bp). The sequences are presented in Table II. They have complementary bases pairs (bold underlined bases) of 2bp, 6bp and 10bp, respectively.

The results in Figure 7A demonstrate that the presence of a short complementary portion of 6bp (Split 1 + Split 2) is optimal to provide an extra free energy contribute resulting in optimal reconfiguration of the complex (first, second and third oligonucleotide), in the presence of the antibody Anti-DIG Ab at 100 nM.

The results in Figure 7B show that the reconfiguration of the complex occurs only in presence of the Anti-DIG antibody. The kinetic profiles have been obtained by adding 100 nM of Anti-DIG Ab in a solution containing all the sensing elements compared to the kinetic profile of the reaction mixture in the absence of Ab.

In Figure 7C, fluorescence trans-cleavage assays have been performed by adding different concentration of Anti-DIG Ab (0.1, 0.5, 1, 5, 10, 50, 75 and 100 nM. A binding curve is shown on Figure 7C.

Figure 7D shows a specificity assay performed using different IgG Ab (anti-DIG, Anti-MUC, Anti-HA, Anti-DIG Fab, Anti-DNP, and Cetuximab). In the assay, the second and third nucleotides are Split 1 + Split 2 respectively, having the recognition element (5) DIG (see Table II). Figure 7D shows that the only antibody detected is Anti-DIG demonstrating the specificity of the Cas-12 based antibody detection system.

As a control experiment, the assay has also been performed in the presence of one Split sequence to confirm that the sensing works only in the presence of the second and third oligonucleotide (Split 1 + Split 2) co-localized on the same target antibody.

Example 6: Detection of the antibody anti-MUC antibody using a Cas-12 based antibody detection system

Fluorescence trans-cleavage assays have been performed according to the protocol described in materials and methods.

In the assay, the second and third nucleotides are Split 1_MUC and Split 2_MUC respectively, having the recognition element (5) MUC (see Table II), a specific peptide (N-ter APDTRPAPGSTAPPA C-ter) with high affinity for anti-MUC Ab in order to match with the specific antibody counterpart.

In Figure 8A, fluorescence trans-cleavage assays have been performed by adding different concentration of Anti-MUC Ab (0.1, 0.5, 1, 5, 10, 50, 75 and 100 nM). A binding curve is shown on Figure 8A.

Figure 8B shows a specificity assay performed using different IgG Ab (anti-MUC, Anti-DIG, Anti-HA, Anti-DIG Fab, Anti-DNP, and Cetuximab). Figure 8B shows that the only antibody detected is Anti-MUC demonstrating the specificity of the Cas-12 based antibody detection system.

Example 7: Detection of the antibody anti-HA antibody using a Cas-12 based antibody detection system

Fluorescence trans-cleavage assays have been performed according to the protocol described in materials and methods.

In the assay, the second and third nucleotides are Split 1_HA and Split 2_HA respectively, having the recognition element (5) HA (see Table II), a specific peptide (YPYDVPDYA) with high affinity for anti-HA Ab in order to match with the specific antibody counterpart.

In Figure 9A, fluorescence trans-cleavage assays have been performed by adding different concentration of Anti-HA Ab (0.1, 0.5, 1, 5, 10, 50, 75 and 100 nM). A binding curve is shown on Figure 9A.

Figure 9B shows a specificity assay performed using different IgG Ab (Anti-HA, anti-MUC, Anti-DIG, Anti-DIG Fab, Anti-DNP, and Cetuximab). Figure 9B shows that the only antibody detected is Anti-HA demonstrating the specificity of the Cas-12 based antibody detection system.

**Claims**

1. A detection system for detecting the presence of a target molecule (13) in a sample comprising

    a) a first oligonucleotide comprising a first hairpin structure comprising

        i. a first 5' single stranded overhang region (2) comprising at least 5 nucleotides;
        ii. a first double stranded stem region (1) comprising at least 16 base pairs, and 0 mismatch or at least 1 mismatch, and wherein said first double stranded stem region (1) comprises a target nucleotides sequence complementary to a guide sequence of a crRNA (11) of a CRISPR-Cas system; and
        iii. a first single stranded loop region (3) comprising from 1 to 4 nucleotides complementary to a protospacer-adjacent motif (PAM) capable of being recognized by the CRISPR-Cas system;

    b) a second oligonucleotide, comprising

        i. a second 5' single stranded region (4) comprising the PAM capable of being recognized by the CRISPR-Cas system;
        ii. a second single stranded region (6) complementary to the target nucleotides sequence of the first double stranded stem region (1); and
        iii. a second single stranded region (7) linked to a terminal recognition element (5) capable of being recognized by the target molecule (13);

    and
    c) a third oligonucleotide, comprising

        i. a third 5' single stranded region (9) linked to a terminal recognition element (5) capable of being recognized by the target molecule (13), wherein said third 5' single stranded region (9) is partially complementary to the second single-stranded region (7); and
        ii. a third single stranded region (8) complementary to the first 5' single-stranded overhang region (2).

2. The detection system according to claim 1, wherein the recognition element (5) is selected from the group comprising a small molecule, a peptide and/or a protein.

3. The detection system according to any one of claims 1-2, wherein the terminal recognition element (5) of the second and third oligonucleotides are identical or different.

4. The detection system according to any one of the preceding claims, further comprising

    d) a CRISPR-Cas system comprising a Cas enzyme (10), and the crRNA (11) comprising a guide sequence complementary to the first double stranded stem region (1); and/or
    e) a DNA reporter construct (12).

5. The detection system according to any one of the preceding claims, wherein the Cas enzyme (10) is selected from the group comprising Cas12, Cas φ and/or Cas 14.

6. The detection system according to any one of the preceding claims, wherein the sequence of the first single stranded loop region (3) of the first oligonucleotide is selected from the group comprising 5'-CAAA-3'; 5'-CAAG-3'; 5'-CAGA-3'; 5'-CGAA-3'.

7. The detection system according to any one of the preceding claims, wherein the sequence of the first oligonucleotide is selected from the group comprising SEQ ID NO: 1, 2, 3, 4, 5, 6, 7, 8, 9, and/or 10.

8. The detection system according to any one of the preceding claims, wherein the sequence of the second oligonucleotide is selected from the group comprising SEQ ID NO; 15 and/or 17.

9. The detection system according to any one of the preceding claims, wherein the sequence of the third oligonucleotide is selected from the group comprising SEQ ID NO: 16, and /or 18.

10. The detection system according to any one of the preceding claims, wherein the guide sequence of the crRNA (11) is SEQ ID NO: 21.

11. A diagnostic device comprising at least one detection system according to any one of the claims 4-10, wherein the DNA reporter construct (12) is integrated on a screen-printed electrode.

12. Use of a detection system according to any one of the claims 1-10 or a diagnostic device according to claim 11 as a diagnostic agent.

13. A kit comprising

   a) a detection system according to any one of the claims 1-10 or a diagnostic device according to claim 11; and
   b) instructions for use to detect the presence of a target molecule (13) in a sample.

14. A method for detecting a target molecule (13) in a sample, the method comprising:

   a) providing a first oligonucleotide comprising a first hairpin structure comprising

      i. a first 5' single stranded overhang region (2) comprising at least 5 nucleotides;
      ii. a first double stranded stem region (1) comprising at least 16 base pairs, and 0 mismatch or at least 1 mismatch, and wherein said first double stranded stem region (1) comprises a target nucleotides sequence complementary to a guide sequence of a crRNA (11) of a CRISPR-Cas system; and
      iii. a first single stranded loop region (3) comprising from 1 to 4 nucleotides complementary to a protospacer-adjacent motif (PAM) capable of being recognized by the CRISPR-Cas system;

   b) providing a second oligonucleotide, comprising

      i. a second 5' single stranded region (4) comprising the PAM capable of being recognized by the CRISPR-Cas system;
      ii. a second single stranded region (6) complementary to the target nucleotides sequence of the first double stranded stem region (1); and
      iii. a second single stranded region (7) linked to a terminal recognition element (5) capable of being recognized by the target molecule (13);

   c) providing a third oligonucleotide, comprising

      i. a third 5' single stranded region (9) linked to a terminal recognition element (5) capable of being recognized by the target molecule (13), wherein said third 5' single stranded region (9) is partially complementary to the second single-stranded region (7); and
      ii. a third single stranded region (8) complementary to the first 5' single-stranded overhang region (2);

   d) providing a sample comprising the target molecule (13);
   e) incubating said sample comprising the target molecule (13) with the first oligonucleotide, the second oligonucleotide, and the third oligonucleotide;
   thereby, in the presence of the target molecule (13) that binds to the terminal recognition element (5) of the second single-stranded region (7) and third 5' single stranded region (9), allowing said second oligonucleotide and said third oligonucleotide to bind to the first oligonucleotide, by triggering its reconfiguration and to form a complex;
   f) incubating the reaction from step e) with

    i. a CRISPR-Cas system comprising a Cas enzyme (10), and the crRNA (11) comprising a guide sequence complementary to the first double stranded stem region (1); and
    ii. a DNA reporter construct (12);

thereby, allowing the guide sequence of the crRNA (11) to bind to the complex of step e), thereby allowing the Cas enzyme (10) to cleave the reporter construct (12); and
g) determining the presence of the target molecule (13) in the sample by measuring signal gain values.

15. The method according to claim 14, wherein steps e) and f) are simultaneous.

16. The method according to claim 14 or 15, wherein step g) is performed using an optical, an electrochemical, a chemiluminescent and/or an electro-chemiluminescent detection system.

17. The method according to any one of the claims 14 to 16, wherein the DNA reporter construct (12) is selected from the group comprising a fluorophore quencher stem-loop oligonucleotide, a methylene blue stem-loop oligonucleotide, and/or a ferrocene stem-loop oligonucleotide immobilized on an electrode.

18. The method according to any one of the claims 14 to 17, wherein step e) and/or f) are performed at a temperature of 15°C to 45°C, preferably, 37°C.

**FIG.1**

**FIG. 2A**

**FIG. 2B**

**FIG. 3A**

**FIG. 3B**

**FIG. 4A**

**FIG. 4B**

FIG.5

Second oligonucleotide   Third oligonucleotide

FIG.6

FIG. 7A

FIG. 7B

FIG. 7C

FIG. 7D

FIG. 8A

FIG. 8B

FIG. 9A

FIG. 9B

Europäisches
Patentamt
European
Patent Office
Office européen
des brevets

# EUROPEAN SEARCH REPORT

Application Number

EP 22 21 6339

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ZHANG JUNYI ET AL: "Target-triggered catalytic hairpin assembly activation of CRISPR/Cas12a for amplified detection of therapeutic monoclonal antibody", SENSORS AND ACTUATORS B: CHEMICAL, ELSEVIER BV, NL, vol. 372, 28 August 2022 (2022-08-28), XP087187805, ISSN: 0925-4005, DOI: 10.1016/J.SNB.2022.132578 [retrieved on 2022-08-28] * Scheme I * | 1-18 | INV. C12Q1/6818 C12Q1/6804 |
| A,D | WO 2020/220013 A1 (UNIV ARIZONA STATE [US]) 29 October 2020 (2020-10-29) * figures 2B, 3A * | 1-18 | |
| A | TANG YANAN ET AL: "A CRISPR-based ultrasensitive assay detects attomolar concentrations of SARS-CoV-2 antibodies in clinical samples", NATURE COMMUNICATIONS, vol. 13, no. 1, 9 August 2022 (2022-08-09), XP093054439, DOI: 10.1038/s41467-022-32371-4 Retrieved from the Internet: URL:https://www.nature.com/articles/s41467-022-32371-4.pdf> * figure 1 * | 1-18 | TECHNICAL FIELDS SEARCHED (IPC) C12Q |

The present search report has been drawn up for all claims

| Place of search | Date of completion of the search | Examiner |
|---|---|---|
| The Hague | 14 June 2023 | Aslund, Fredrik |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 21 6339

This annex lists the patent family members relating to the patent documents cited in the above-mentioned European search report.
The members are as contained in the European Patent Office EDP file on
The European Patent Office is in no way liable for these particulars which are merely given for the purpose of information.

14-06-2023

| Patent document cited in search report | Publication date | Patent family member(s) | Publication date |
|---|---|---|---|
| WO 2020220013 A1 | 29-10-2020 | NONE | |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2020030032 W

**Non-patent literature cited in the description**

- **BARBER K. W.** *Molecular Cell,* 2021, vol. 81 (17), 3650-8